# EUROPÄISCHE PATENTSCHRIFT

(11) **EP 4 203 663 B1**
(45) Veröffentlichungstag und Bekanntmachung des Hinweises auf die Patenterteilung: **27.08.2025**
(21) Anmeldenummer: 21772736.1
(22) Anmeldetag: 31.08.2021
(51) Int. Cl.: A01B 39/18, A01B 79/00, A01M 21/02

(54) **VERFAHREN ZUR ERKENNUNG VON STÖRUNGEN DER MECHANISCHEN AUTOMATISIERTEN ENTFERNUNG VON UNKRÄUTERN**
METHOD FOR DETECTING FAULTS IN THE MECHANICAL AUTOMATED REMOVAL OF WEEDS
PROCÉDURE DE DÉTECTION DES PANNES DE L'ÉLIMINATION MÉCANIQUE AUTOMATISÉE DES MAUVAISES HERBES

(30) Priorität: 31.08.2020 DE 102020210951
(43) Veröffentlichungstag der Anmeldung: 05.07.2023
(73) Patentinhaber: Amazonen-Werke H. Dreyer SE & Co. KG, 49205 Hasbergen (DE)
(72) Erfinder: WILD, Karl, 01454 Ullersdorf (DE)
(86) Internationale Anmeldenummer: PCT/EP2021/073935
(87) Internationale Veröffentlichungsnummer: WO 2022/043568

(56) Entgegenhaltungen:
- EP-B1- 3 192 342
- WO-A1-2020/259970
- CN-A- 108 135 122
- CN-A- 108 508 889
- CN-A- 110 024 555
- CN-U- 209 560 367
- CN-U- 209 903 226
- IT-A1- UB20 155 305
- US-A1- 2011 211 733
- US-A1- 2018 153 084
- US-A1- 2019 200 510

## Beschreibung

Die Erfindung betrifft ein Verfahren zur Erkennung von Störungen der mechanischen automatisierten Entfernung von Unkräutern bei der Feldbearbeitung von auf einem Feld wachsenden Kulturpflanzen mit einem mobilen Hackgerät.

Gegenwärtig wird die Durchführung von Feldarbeiten in den meisten Fällen durch eine Traktor-Geräte-Kombination durchgeführt. Der Fahrer steuert dabei den Traktor und überwacht den Arbeitsprozess, der vom angehängten oder angebauten Gerät erledigt wird. Mittlerweile gibt es auch erste Lösungen, bei denen mit autonomen Roboterfahrzeugen Arbeiten erledigt werden können. Ein Fahrzeugfahrer wird dabei nicht mehr benötigt. CN 108 508 889 offenbart solch eine Lösung. Diese Verfahren werden in Zukunft erheblich an Bedeutung gewinnen. Am häufigsten wird momentan diese neue Technik zur mechanischen Unkrautbekämpfung mit Hackgeräten eingesetzt. Unter optimalen Bedingungen werden gute Arbeitsergebnisse erzielt. Leider sind diese nicht allzu oft gegeben, so dass es zu Störungen bei der Hackarbeit kommen kann. Dazu zählen z.B. Verstopfungen an den Hackwerkzeugen, Unkraut bleibt an den Hackwerkzeugen hängen oder Steine klemmen sich ein. Diese Störungen können nicht nur zu einer Reduzierung des Bekämpfungserfolges führen, sondern auch zu erheblichen Schäden an den Kulturpflanzen. Bei der üblichen Traktor-Hackgerät-Kombination nimmt der Fahrer diese Störungen wahr und kann sie beseitigen. Bei den Hackrobotern gibt es momentan keine geeigneten Systeme zur Störungserkennung. Manche Hackroboter sind zwar mit einer Kamera ausgestattet, die Bilder auf das Smart Phone des Bedieners senden kann. Der Bediener kann aber nicht ständig den Hackprozess über einen Bildschirm beobachten, denn durch diese Bindung ginge der Vorteil des fahrerlosen Hacksystems größtenteils verloren. Von daher ist eine Technik gefragt, die Störungen erfasst und dann den Bediener eine entsprechende Information zuschicken kann. Dann kann der Benutzer anreisen und die Störung beseitigen. Noch günstiger wäre es, wenn die Hackmaschine ohne ein Zutun des Betreuers die Störung eigenständig beseitigen könnte.

Durch die Bewegung der Werkzeuge im Boden werden die Werkzeuge in Schwingung versetzt. Die Amplitude und Frequenz dieser Schwingungen hängt von einer Reihe von Parametern ab, wie z.B. spezifischer Bodenwiderstand, Arbeitstiefe, Fahrgeschwindigkeit und natürlich vom Werkzeug selbst. Kommt es nun zu einer Verstopfung durch Erde oder Unkraut, die sich vor dem Werkzeug aufschiebt, so ändert sich das Schwingungsverhalten im Hinblick auf Amplitude und Frequenz. Gleiches gilt auch für ein Einklemmen eines Steines oder wenn sich Unkraut um das Werkzeug wickelt (bei rotatorisch arbeitenden Werkzeugen) oder am Werkzeug hängen bleibt oder das Werkzeug/Schar sich verbiegt oder verloren geht. Wird nun ein Beschleunigungssensor, idealerweise ein 3-achsiger, am Werkzeug angebracht, so können diese Schwingungen gemessen und an eine zentrale Einheit zur Erfassung und Verarbeitung weitergereicht werden. Durch entsprechende Signalanalysetechniken (FFT, digitale Filter etc.) können die Veränderungen im Schwingungsverhalten registriert und somit Probleme erkannt werden. Zum Beginn der Hackarbeit kann zunächst auf den ersten ca. 100 m eine Kalibrierung durchgeführt werden, das heißt es werden die Messwerte bei störungsfreien Ablauf erfasst. Die Messwerte werden einer Filterung unterzogen bzw. geglättet und es werden Schwellenwerte (Unter- und Obergrenzen für Frequenzen und Amplituden) für die unterschiedlichen Schwingungsebenen erstellt. Für jedes Hackwerkzeug entsteht dadurch eine Signatur. Werden nun beim Hacken für längere Zeit die Schwellenwerte über bzw. unterschritten, so ist davon auszugehen, dass eine Störung vorliegt. Die Maschine stoppt selbständig und der Landwirt kann mittels Nachrichtenübertragung, z.B. per SMS informiert werden.

Bei der Erfassung und Auswertung der Schwingungen an einem Hackgerät kann es aber zu Problemen kommen, indem sich Schwingungen überlagern und/oder eine andere Art der Schwingungserregung auftreten kann, die mit der eigentlichen Hackbearbeitung nichts zu tun hat. Dies können beispielsweise Schwingungen sein, die infolge von am Hackgerät während der Fahrt auftretenden Beschleunigungen generiert oder das Anstoßen eines Werkzeugs an einen Stein oder eine Änderung der Bodenart hervorgerufen werden. Eine so durchgeführte Störungsüberwachung ist noch zu stark fehlerbehaftet.

Es ist daher Aufgabe der Erfindung, Möglichkeiten für eine verbesserte und sichere Erkennung von Störungen, die bei der mechanischen autonomen Entfernung von Unkräutern bei der Feldbearbeitung von Kulturpflanzen auftreten können.

Erfindungsgemäß wird diese Aufgabe mit einem Verfahren, das die Merkmale des Anspruchs 1 aufweist, gelöst. Vorteilhafte Ausführungsformen und Weiterbildungen der Erfindung können mit in untergeordneten Ansprüchen bezeichneten Merkmalen realisiert werden.

Bei dem erfindungsgemäßen Verfahren werden bei
einem erfindungsgemäßen Schritt i) mit mindestens einem im Bereich eines Werkzeugs am Hackgerät angeordneten Ultraschallabstandssensor Ultraschallwellen senkrecht in Richtung Erdboden emittiert und der Abstand zur Erdbodenoberfläche durch Bestimmung der Laufzeit der emittierten und vom Erdboden reflektierten Ultraschallwellen bis zu deren Detektion bestimmt
   und/oder
bei einem nicht erfindungsgemäßen Schritt ii) wird mit mindestens einer Strahlungsquelle, die eine Lichtquelle oder auch die Sonne sein kann, elektromagnetische Strahlung in Richtung des Erdbodens in einen Oberflächenbereich emittiert, in dem eine mechanische Unkrautentfernung durchgeführt wird oder werden soll und die Intensität von von dort reflektierter oder gestreuter elektromagnetischer Strahlung mit mindestens einem am Hackgerät angeordneten optischen Detektor erfasst und mit der erfassten Intensität bestimmt wird, ob eine ausreichende Bodenbearbeitung erfolgt ist, die zu einer Entfernung von Unkraut geführt hat und/oder eine zu große Staubfreisetzung erfolgt ist und/oder
in einem nicht erfindungsgemäßen Schritt iii) werden an einzelnen Werkzeugen mit Dehnungsmesstreifen Scher- und/oder Biegespannungen detektiert, die mit Kalibrierwerten, die bei dem entsprechenden Werkzeug bei einer korrekten Unkrautentfernung ermittelt wurden, verglichen und bei der Überwachung berücksichtigt werden und/oder
in einem nicht erfindungsgemäßen Schritt iv) Luftschallwellen, die infolge der Unkrautentfernung mit mindestens einem Werkzeug emittiert werden, mit mindestens einem Mikrofon detektiert und die erfassten Luftschallspektren mit einer elektronischen Auswerteeinheit mit vorab bei einer fehlerfreien Unkrautentfernung erfassten Luftschallspektren verglichen werden.

Die Luftschallwellen können dabei frequenzaufgelöst erfasst oder ausgewertet werden. Es besteht auch die Möglichkeit, bestimmte typische Frequenzen oder Frequenzbereiche durch Filterung für die Auswertung zu nutzen.

Die emittierten Luftschallwellen oder Schwingungen sind auch von der jeweiligen Bodenart, Bodenfeuchte, Humusgehalt, Fahrgeschwindigkeit, Arbeitstiefe oder den jeweiligen Werkzeug abhängig. Da die Hackarbeit und das Ergebnis auch von diesen Parametern abhängig sind, können über eine Luftschallwellen- oder Schwingungsanalyse Rückschlüsse über das Hackgerät bzw. die Hackarbeit gezogen werden.

Zusätzlich können an den einzelnen Werkzeugen mit Detektoren Schwingungen bezüglich ihrer Amplitude und frequenzaufgelöst und/oder Drehzahlen von rotierenden Elementen an Werkzeugen erfasst und für eine Funktionalitätsüberwachung berücksichtigt werden. Für eine Detektion von Schwingungen kann man an sich bekannte Ultraschallwellenwandler oder Beschleunigungssensoren einsetzen. Mittels dieser Schwingungsanalyse, die prinzipiell bekannt war kann man die Überwachungsgenauigkeit in Verbindung mit der gemeinsamen Durchführung mindestens eines der Verfahrensschritte i), ii) und/oder iii) verbessern.

Bei trockenen Bedingungen kann es zu einer mehr oder weniger starken Staubfreisetzung kommen und eine unerwünschte Staubablagerung auf den Kulturpflanzen stattfinden. Mit einem preiswerten optischen Detektor kann die Staubentwicklung ermittelt werden und bei einer zu hohen Staubfreisetzung kann über ein entsprechendes Regelungssystem die Fahrgeschwindigkeit reduziert werden. Dabei kann man die Staubfreisetzung prinzipiell gemäß dem Verfahrensschritt ii) bestimmen. Es besteht dabei auch die Möglichkeit, die jeweilige(n) Intensität(en) lediglich für bestimmte Wellenlängen zu berücksichtigen.

Vorteilhaft sollten an allen Werkzeugen zu gleichen Zeitpunkten erfasste gleichartige Messwerte miteinander verglichen werden. Damit kann man zumindest größere Unterschiede feststellen, die zumindest auf einen Defekt oder eine Fehlfunktion an einem oder mehreren Werkzeug(en) des jeweiligen Hackgeräts hinweisen können.

Vor Beginn einer Überwachung kann eine Kalibrierfahrt und/oder während der Bearbeitung mindestens eine Kalibrierfahrt durchgeführt werden, bei der Messwerte erfasst werden, die bei einer funktionsfähigen zumindest nahezu störungsfreien Bearbeitung erfasst worden sind. Diese bei einer Kalibrierfahrt erfassten Messwerte können als Referenz für nachfolgend bei der Bearbeitung erfasste Messwerte genutzt werden. Mit während der Bearbeitung erfassten Kalibriermesswerten kann man beispielsweise sich verändernde äußere Randbedingungen, wie z.B. eine höhere Bodenverdichtung und/oder Feuchtigkeit kompensieren bzw. berücksichtigen.

Außerdem können Kamerabilder vom Hackgerät übertragen werden, so dass der Landwirt erkennen kann, welche Art von Störung vorliegt. Der Landwirt kann dann entsprechend reagieren, z.B. durch Anreise und Störungsbehebung oder Auslösung einer Störungsbehebungsmaßnahme, die vom Hackgerät selbsttätig durchgeführt werden kann.

Bestimmte Störungen lassen sich aus den verarbeiteten Messdaten herauslesen. Dementsprechend kann dann in bestimmten Fällen die Maschine selbständig eine Störungsbeseitigungsprozedur starten, wie z.B. bei Verstopfungen ein Ausheben des Hackgerätes und nach kurzer Fahrt (ca. 1 m) ein wieder Absenken durchgeführt werden. Auch können, bevorzugt im ausgehoben Zustand, durch eine technische Einrichtung Schwingungen in ein Werkzeug oder dessen Umgebung am Hackgerät mit einer Amplitude eingekoppelt werden, bei der eine Freisetzung einer Verstopfung oder ein Verklemmen von Unkraut oder Gegenständen vom jeweiligen Werkzeug erreicht werden kann. So kann z.B. mit einem Verschieberahmen oder einer Unwuchteinrichtung das Hackgerät geschüttelt werden, so dass anhängendes Unkraut etc. abgeschüttelt werden kann. Als eine Einrichtung zur Einkopplung von Schwingungen kann eine rotierende Masse mit einer Unwucht eingesetzt werden.

Auch beim Abschütteln können Messdaten erfasst werden, so dass durch die Datenanalyse erkennbar wird, wie sich das Schwingungsverhalten der Werkzeuge verändert. Dadurch kann zum einen die Störungsanalyse verbessert und zum anderen die Beseitigung der Störung erkannt werden.

Auch bei Wendevorgängen am Feldende können Schwingungen und andere Messwerte an den Hackwerkzeugen erfasst werden. Dazu kann es notwendig sein, kurz anzuhalten. Damit kann festgestellt werden, ob Veränderungen an den Werkzeugen stattgefunden haben.

Kalibrierfahrten, wie schon vorher erläutert, können auch zwischendurch durchgeführt werden. Hierzu ist es günstig, wenn der Landwirt diese über die Videoübertragung beobachtet, damit sichergestellt wird, dass diese Kalibrierfahrt störungsfrei verläuft.

Es kann mindestens eine Kamera am Hackgerät vorhandenen sein. Deren erfasste Signale können an eine elektronische Bildauswerteeinheit übermittelt werden. Mit den mit der elektronischen Bildauswerteeinheit ausgewerteten Daten kann überwacht werden, ob Unkrautpflanzen nach einem Verschütten ausreichend mit Erde überdeckt worden sind und/oder ob Kulturpflanzen beschädigt worden sind.

Bei der mechanischen Unkrautbekämpfung gibt es nämlich im Wesentlichen drei verschiedene Maßnahmen: Abschneiden, Herausreißen oder Verschütten. Am effektivsten dabei ist Herausreißen oder Verschütten. Um nun den Bekämpfungserfolg dieser beiden Maßnahmen jeweils ermitteln zu können, kann die Ermittlung des Unkrautbedeckungsgrades herangezogen werden. Dieser kann mit Hilfe einer Kamera und elektronischen Bildauswerteeinheit bestimmt werden. Es kann dabei das nichtbedeckte sichtbare Unkraut oder Teile davon vor und nach der Maßnahme ermittelt werden. Nach dem Verschütten soll der Unkrautbedeckungsgrad null sein, d.h. es ist kein Unkraut mehr sichtbar. Wird hingegen Herausgerissen, dann soll er möglichst groß sein, d.h. das Unkraut liegt vollständig und "langgestreckt" auf der Bodenoberfläche, so dass es schnell und gut verdörrt und dann abstirbt.

Bei Hackarbeiten dürfen die Kulturpflanzen nicht oder nur minimal beschädigt werden. Die Kulturpflanzen sollten nach dem Einsatz des Hackgerätes so aussehen wie vor dem Hacken. Dies kann ebenfalls mit Hilfe von Kameras und einer elektronischen Bildauswerteeinrichtung ermittelt werden. Die Kameras sollten dazu vor und nach den Werkzeugen am Hackgerät angeordnet sein. Durch einen einfachen Vergleich der Kulturpflanzenbilder vor und nach dem Hacken kann man erkennen, in wie weit sich an den Kulturpflanzen etwas verändert hat bzw. ob eine Schädigung verursacht wurde.

Mit einem elektronischen Regelsystem kann/können bei einem Über- oder Unterschreiten von mindestens einem vorgegebenen Schwellwert eines erfassten Messsignales die Fahrgeschwindigkeit, die Arbeitstiefe und/oder die Abstände der Werkzeuge zueinander angepasst werden.

Es besteht auch die Möglichkeit, die für die Fortbewegung des Hackgerätes erforderliche Kraft und/oder Leistung zu bestimmen und damit die Arbeitstiefe der Werkzeuge zu beeinflussen und/oder die Fahrgeschwindigkeit an Bodenverhältnisse lokal definiert anzupassen. Bei Kenntnis der eingestellten Arbeitstiefe der Werkzeuge können dann Rückschlüsse über jeweilige Bodenart und Bodendichte getroffen werden, die helfen die Messdaten besser zu interpretieren und auch Auskunft über die Schneidenschärfe von Werkzeugen zu geben.

In den meisten Fällen treten Störungen nicht gleichzeitig an mehreren Werkzeugen eines Hackgeräts auf, sondern meistens nur an einem oder zwei. Deshalb ist es vorteilhaft, dass die gleichzeitig erfassten Messwerte, die im Bereich aller einzelnen Werkzeuge eines Hackgeräts erfasst werden, ständig miteinander verglichen werden, da dadurch ein gestörtes Werkzeug schnell identifiziert werden kann.

Wenn die Regelung für die Hackwerkzeugführung nicht richtig funktioniert oder die Werkzeuge nicht richtig eingestellt sind und dann (größere) Kulturpflanzen umgehackt werden, dann ergeben sich in den Messdaten rhythmisch auftretende Signale (v.a. bei größeren/stabileren Kulturpflanzen). Diese können herangezogen werden, um eine fehlerhafte Führung oder Einstellung der Hackwerkzeuge zu erkennen.

Um nicht zu jedem Sensor eine Kabelverbindung für Stromversorgung und Datentransfer legen zu müssen, können auch Systeme verwendet werden, die mit Energy Harvesting die Energieversorgung gewährleisten (z.B. durch sich bewegende Werkzeuge) und den Datentransfer drahtlos ermöglichen (WLAN; Bluetooth etc.).

Bei rotatorisch arbeitenden Werkzeugen können auch Drehzahlsensoren eingesetzt werden. Deren Signale können entsprechend ausgewertet werden, wobei auch hier der Vergleich der Signale von den einzelnen Werkzeugen schnell zu einer Erkennung von Störungen an einem Werkzeug führen kann.

Erfindungsgemäß ist der Einsatz von Ultraschallabstandssensoren (US-Sensoren) vorgesehen. Verstopfungen oder anhängendes Unkraut führt zu einer Anhäufung von Erde oder Pflanzenmaterial. Vertikal angeordnete US-Sensoren können diese Anhäufungen ermitteln und dementsprechende Störungen erkennen. Datenerfassungen und Analysen (Kalibrierung etc.) werden dann wie weiter oben schon vorgestellt, durchgeführt. Auch können die US-Sensoren hinter den Hackwerkzeugen feststellen, ob gerade größeres Unkraut auch "flach liegt". Es gibt auch Hackwerkzeuge, die Häufeln, d.h. das Unkraut mit Erde zudecken. Hier kann mit US-Sensoren festgestellt werden, ob die Häufelschicht ausreichend hoch ist und ob Unkraut noch aus der angehäufelten Erde herausragt. Mit dieser Technik kann aber auch festgestellt werden, ob auch Kulturpflanzen in unerwünschter Weise zugedeckt wurden.

Die Hackwerkzeugüberwachung kann um zusätzliche Systeme zur Qualitätsüberprüfung der Hackarbeit erweitert werden. Typischerweise ist der Boden vor dem Hackwerkzeug relativ glatt. Nach einem ordnungsgemäßen Hacken ist die Oberfläche relativ rau, weil die Oberfläche aufgebrochen wurde. Mit Hilfe einer Strahlungsquelle, die elektromagnetische Strahlung emittiert, und mindestens einem optischen Detektor kann dieses Rauigkeit erfasst werden. Die Strahlungsquelle emittiert die elektromagnetische Strahlung in Richtung des Erdbodens, von dort wird die elektromagnetische Strahlung mehr oder weniger gut reflektiert und gestreut und reflektierte und gestreute elektromagnetische Strahlung trifft auf mindestens einen optischen Detektor, der am Hackgerät angeordnet ist, auf. Je rauer die Bodenoberfläche ist, desto mehr ist die reflektierte und am Boden gestreute elektromagnetische Strahlung diffus und die Intensität der mit dem einen optischen Detektor erfassten reflektierten und gestreuten elektromagnetischen Strahlung ist entsprechend geringer, als bei einem unbearbeiteten Boden. Werden mehrere optische Detektoren im Bereich eines Werkzeugs angeordnet, kann auch ausgewertet werden, ob lediglich ein einzelner optischer Detektor oder eine größere Anzahl optischer Detektoren elektromagnetische Strahlung mit einer Intensität erfassen, die einen vorgegebenen Schwellwert überschreitet. Dabei kann man ab einer bestimmten Anzahl der mehreren optischen Detektoren, bei denen gleichzeitig dieser Schwellwert überschritten wird, feststellen, dass eine ausreichende Bearbeitung erfolgt ist.

Ist der Boden zu feucht, so entstehen in unerwünschter Weise Kluten, die die Rauigkeit stark zunehmen lassen. Diese ermittelte zu starke Rauigkeit dient dann als Auslöser zum Beenden der Hackarbeit oder zur Verringerung der Arbeitstiefe.

Alle erfassten Messwerte bzw. -signale können in einer elektronischen Datenanalyseeinrichtung ausgewertet und mit vorteilhaft darin gespeicherten Kalibriermess- oder Referenzwerten verglichen werden. Dabei können auch vorgegebene Schwellwerte für jeweilige Messwerte bzw. Messsignale in der elektronischen Datenanalyseeinrichtung berücksichtigt werden.

Die Erfindung beruht auf den Einsatz entsprechender Sensorik an den Werkzeugen bzw. Zinken eines Hackgerätes und einer dazugehörigen elektronischen Datenanalyseeinrichtung sowie ggf. einer geeigneten Aktorik.

## Patentansprüche

1. Verfahren zur Erkennung von Störungen der mechanischen automatisierten Entfemung von Unkräutern bei der Feldbearbeitung von auf einem Feld wachsenden Kulturpflanzen mit einem mobilen Hackgerät,
wobei mit mindestens einem im Bereich eines Werkzeugs am Hackgerät angeordneten Ultraschallabstandssensor Ultraschallwellen senkrecht in Richtung Erdboden emittiert und der Abstand zur Erdbodenoberflächen durch Bestimmung der Laufzeit der emittierten und vom Erdboden reflektierten Ultraschallwellen bis zu deren Detektion bestimmt wird und dementsprechende Störungen erkannt werden.

2. Verfahren nach Anspruch 1, **dadurch gekennzeichnet, dass** an allen Werkzeugen zu gleichen Zeitpunkten erfasste gleichartige Messwerte miteinander verglichen werden.

3. Verfahren nach einem der vorhergehenden Ansprüche, **dadurch gekennzeichnet, dass** vor Beginn einer Überwachung eine Kalibrierfahrt und/oder während der Bearbeitung mindestens eine Kalibrierfahrt durchgeführt wird.

4. Verfahren nach einem der vorhergehenden Ansprüche, **dadurch gekennzeichnet, dass** mit mindestens einer Kamera Bereiche an den Werkzeugen überwacht und die erfassten Bilder bei einer mit einem Detektor erfassten Fehlfunktion an eine Überwachungszentrale übermittelt werden, so dass Maßnahmen zur Störungsbehebung initiiert werden können.

5. Verfahren nach einem der vorhergehenden Ansprüche, **dadurch gekennzeichnet, dass** an Werkzeugen jeweils eine Einrichtung vorhanden ist, mit der bei Erfassung einer Fehlfunktion bei dem in eine Ruhestellung gebrachten Werkzeug Schwingungen in das jeweilige Werkzeug oder dessen Umgebung am Hackgerät mit einer Amplitude eingekoppelt werden, bei der eine Freisetzung einer Verstopfung oder ein Verklemmen von Unkraut oder Gegenständen vom jeweiligen Werkzeug erreicht wird.

6. Verfahren nach dem vorhergehenden Anspruch, **dadurch gekennzeichnet, dass** als Einrichtung zur Einkopplung von Schwingungen eine rotierende Masse mit einer Unwucht eingesetzt wird.

7. Verfahren nach einem der vorhergehenden Ansprüche, **dadurch gekennzeichnet, dass** bei Verstopfungen ein Ausheben des Hackgerätes und nach kurzer Fahrt ein wieder Absenken durchgeführt werden.

8. Verfahren nach einem der vorhergehenden Ansprüche, **dadurch gekennzeichnet, dass** mit mindestens einer am Hackgerät vorhandenen Kamera, deren erfasste Signale an eine elektronische Bildauswerteeinheit übermittelt werden und den mit der elektronischen Bildauswerteeinheit ausgewerteten Daten überwacht wird, ob Unkrautpflanzen nach einem Verschütten ausreichend mit Erde überdeckt, aus dem Erdboden herausgerissen worden sind und/oder ob Kulturpflanzen beschädigt worden sind.

9. Verfahren nach einem der vorhergehenden Ansprüche, **dadurch gekennzeichnet, dass** mit einem elektronischen Regelsystem bei einem Über- oder Unterschreiten von mindestens einem vorgegebenen Schwellwert eines erfassten Messsignales die Fahrgeschwindigkeit, die Arbeitstiefe und/oder die Abstände der Werkzeuge zueinander angepasst werden.

10. Verfahren nach einem der vorhergehenden Ansprüche, **dadurch gekennzeichnet, dass** die für die Fortbewegung des Hackgerätes erforderliche Kraft und/oder Leistung bestimmt und damit die Arbeitstiefe der Werkzeuge beeinflusst und/oder die Fahrgeschwindigkeit an Bodenverhältnisse lokal definiert angepasst wird/werden.

## Claims

1. Method for detecting disturbances in the mechanical automated removal of weeds during field cultivation of crops growing in a field by means of a mobile hoeing apparatus,
wherein at least one ultrasonic distance sensor arranged in the region of a tool on the hoeing apparatus emits ultrasonic waves perpendicularly in the direction of the soil, and the distance to the soil surface is determined by determining the travel time of the emitted ultrasonic waves reflected by the soil until their detection, and corresponding disturbances are detected.

2. Method according to claim 1, **characterized in that** measured values of the same type which are detected on all tools at the same points in time are compared with one another.

3. Method according to either of the preceding claims, **characterized in that** a calibration run is carried out before monitoring begins and/or at least one calibration run is carried out during the cultivation.

4. Method according to any of the preceding claims, **characterized in that** regions on the tools are monitored by means of at least one camera, and the captured images are transmitted to a monitoring center in the event of a malfunction detected by means of a detector, so that measures for troubleshooting can be initiated.

5. Method according to any of the preceding claims, **characterized in that** a device is provided on each tool, by means of which device, when a malfunction is detected and the tool is brought into a rest position, vibrations are coupled into the relevant tool or its surroundings on the hoeing apparatus with an amplitude at which a release of a blockage or of a jamming of weeds or objects from the relevant tool is achieved.

6. Method according to the preceding claim, **characterized in that** a rotating mass which has an imbalance is used as the device for coupling vibrations.

7. Method according to any of the preceding claims, **characterized in that** in the event of blockages, the hoeing apparatus is lifted up and, after a short drive, lowered again.

8. Method according to any of the preceding claims, **characterized in that** at least one camera provided on the hoeing apparatus, the detected signals of which are transmitted to an electronic image evaluation unit, and the data evaluated by the electronic image evaluation unit are used to monitor whether weeds have been sufficiently covered with soil after being buried, have been pulled out of the soil and/or whether crops have been damaged.

9. Method according to any of the preceding claims, **characterized in that** an electronic control system adjusts the driving speed, working depth and/or distances between the tools when a detected measurement signal exceeds or falls below at least one predetermined threshold value.

10. Method according to any of the preceding claims, **characterized in that** the force and/or power required for the movement of the hoeing equipment are determined and therefore the working depth of the tools is influenced, and/or the driving speed is adjusted to the soil conditions in a locally defined manner.

## Revendications

1. Procédé pour la reconnaissance de perturbations de l'enlèvement mécanique automatisé de mauvaises herbes lors du traitement de champ, à l'aide d'une bineuse mobile, de cultures poussant dans un champ,
dans lequel, à l'aide d'au moins un capteur de distance ultrasonique disposé dans la zone d'un outil sur la bineuse, des ondes ultrasonores sont émises perpendiculairement en direction du sol et la distance par rapport aux surfaces du sol est établie en déterminant le temps de propagation des ondes ultrasonores émises et réfléchies par le sol jusqu'à leur détection, et des perturbations correspondantes sont reconnues.

2. Procédé selon la revendication 1, **caractérisé en ce que** des valeurs de mesure de même type enregistrées aux mêmes moments sur tous les outils sont comparées entre elles.

3. Procédé selon l'une des revendications précédentes,
**caractérisé en ce qu'**un parcours d'étalonnage est effectué avant le début d'une surveillance et/ou au moins un parcours d'étalonnage est effectué pendant le traitement.

4. Procédé selon l'une des revendications précédentes,
**caractérisé en ce que** des zones sur les outils sont surveillées par au moins une caméra et les images enregistrées sont transmises à une centrale de surveillance en cas de dysfonctionnement enregistré par un détecteur, en sorte que des mesures peuvent être lancées pour éliminer la perturbation.

5. Procédé selon l'une des revendications précédentes,
**caractérisé en ce qu'**il existe respectivement un dispositif sur des outils, dispositif à l'aide duquel, en cas d'enregistrement d'un dysfonctionnement dans l'outil mis en position de repos, des vibrations sont injectées dans l'outil respectif ou son environnement sur la bineuse avec une amplitude à laquelle l'on parvient à une libération d'un bourrage ou un coincement de mauvaises herbes ou d'objets par l'outil respectif.

6. Procédé selon la revendication précédente, **caractérisé en ce qu'**une masse rotative présentant un déséquilibre est utilisée comme dispositif pour l'injection de vibrations.

7. Procédé selon l'une des revendications précédentes,
**caractérisé en ce que,** en cas de bourrage, la bineuse est soulevée et, après un bref déplacement, elle est à nouveau abaissée.

8. Procédé selon l'une des revendications précédentes,
**caractérisé en ce que,** à l'aide d'au moins une caméra présente sur la bineuse, caméra dont les signaux enregistrés sont transmis à une unité électronique d'analyse d'images, et à l'aide des données analysées par l'unité électronique d'analyse d'images, l'on surveille si les plantes adventices ont été suffisamment recouvertes de terre après un déversement, si elles ont été arrachées du sol et/ou si les cultures ont été endommagées.

9. Procédé selon l'une des revendications précédentes,
**caractérisé en ce que** la vitesse de déplacement, la profondeur de travail et/ou les distances entre les outils sont adaptées les unes aux autres au moyen d'un système de régulation électronique en cas de dépassement vers le haut ou vers le bas d'au moins une valeur seuil prédéfinie d'un signal de mesure enregistré.

10. Procédé selon l'une des revendications précédentes,
**caractérisé en ce que** la force et/ou la puissance nécessaire pour le déplacement vers l'avant de la bineuse est/sont déterminée(s) et la profondeur de travail des outils est ainsi influencée et/ou la vitesse de déplacement est adaptée aux conditions du sol de manière définie localement.
